# EUROPEAN PATENT SPECIFICATION

(11) **EP 4 188 269 B1**
(45) Date of publication and mention of the grant of the patent: **25.11.2026**
(21) Application number: 21762812.2
(22) Date of filing: 29.07.2021
(51) Int. Cl.: A61B 34/30

(54) **OBJECT DETECTION AND AVOIDANCE IN A SURGICAL SETTING**
OBJEKTERKENNUNG UND -VERMEIDUNG IN EINER CHIRURGISCHEN UMGEBUNG
DÉTECTION ET ÉVITEMENT D'OBJET DANS UNE INSTALLATION CHIRURGICALE

(30) Priority: 31.07.2020 US 202063059317 P
(43) Date of publication of application: 07.06.2023
(73) Proprietor: Mazor Robotics Ltd., 3079567 Caesarea (IL)
(72) Inventor: JUNIO, Dany, 3079567 Caesarea (IL); ELLMAN, Aviv, 3079567 Caesarea (IL); RATZABI, Elad, 3079567 Caesarea (IL); ZUCKER, Ido, 3079567 Caesarea (IL); ZEHAVI, Eli, 3079567 Caesarea (IL); KLEYMAN, Leonid, 3079567 Caesarea (IL)
(74) Representative: Viering, Jentschura & Partner mbB Patent- und Rechtsanwälte
(86) International application number: PCT/IL2021/050918
(87) International publication number: WO 2022/024130

(56) References cited:
- EP-A1- 2 821 024
- US-A1- 2014 276 943
- US-A1- 2019 090 966

## Description

### FIELD

The present disclosure is related generally to robotic surgery, and is more particularly related to maintaining situational awareness while controlling a robot in a surgical setting.

### BACKGROUND

Surgical procedures may be conducted entirely manually, manually but with robotic assistance, or autonomously using one or more robots. During a surgical procedure, the surgical environment may become crowded, with one or more tools, instruments, implants, or other medical devices, one or more arms, hands, and/or figures of one or more physicians or other medical personnel, and/or one or more robotic arms, together with at least a portion of the patient's anatomy, all positioned in or around (and, in some instances, moving into, out of, and/or around) a work volume to carry out the surgical procedure.

From e.g. US 2019 / 090 966 A1 a surgical robotic system is known, comprising: a robot comprising a robotic arm, at least one processor and a memory storing instructions for execution by the at least one processor that, when executed, cause the at least one processor to: use surgical plan comprising first information about a planned position of at least one object relative to a patient's anatomy and second information about a surgical objective and calculate a movement path for the robotic arm based on the first information and the second information.

Further surgical robotic systems are known from e.g. EP 2 821 024 A1 and US 2014 / 276 943 A1. The latter publication discloses a system comprising a localizer with a camera unit configured to track a surgical robot as well as a machine vision system including a 3-dimensional machine vision camera. The machine vision camera is arranged so that a field-of-view of the vision camera encompasses the surgical site and objects in proximity to the surgical site. Via the machine vision camera, the system can identify and track objects of known sizes and shapes, namely a surgical instrument (e.g. a cutting bur), retractor assemblies, a femur and a tibia.

### SUMMARY

The present invention provides a surgical robotic system with the features according to claim 1. Further preferred embodiments of the surgical robotic system are described in the dependent claims. None of the methods disclosed herein form part of the claimed invention.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present invention will become apparent to those skilled in the art upon consideration of the embodiment descriptions provided hereinbelow.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2 is a flowchart of a method according to at least one embodiment of the present disclosure;
Fig. 3 is another flowchart of a method according to at least one embodiment of the present disclosure;
Fig. 4 is another flowchart of a method according to at least one embodiment of the present disclosure;
Fig. 5 is another flowchart of a method according to at least one embodiment of the present disclosure;
Fig. 6A is another flowchart of a method according to at least one embodiment of the present disclosure;
Fig. 6B is another flowchart of a method according to at least one embodiment of the present disclosure;
Fig. 7 is another flowchart of a method according to at least one embodiment of the present disclosure; and
Fig. 8 is another flowchart of a method according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

Orthopedic surgery may require a surgeon to perform high risk intervention actions such as cutting and drilling within very sensitive and delicate surrounding tissue (spinal nerves, main blood vessels), particularly during procedures such as spinal fusion procedures (TLIF, PLIF, XLIF), minimally invasive spinal fusion procedures, bony decompression procedures, and other such procedures. The greater the precision obtained or maintained during such procedures, the better patient safety may be ensured and the greater the likelihood of success of the clinical procedure. Technological solutions such as optical navigation and surgical robotics have been developed to improve precision during such procedures. The surgical treatments described herein do not form part of the present invention but are helpful in understanding the present invention.

When using a robotic system within the surgical working environment, a portion of the robotic system (e.g., a robotic arm) may move near the patient's body to improve accuracy and reduce footprint within the operating room. This potentially introduces a collision risk between the robotic system and the patient or any solid objects within the robotic system's working environment (such as but not limited to bone to robot docking tools or structure, surgical tools, and implants).

Where a robotic system does not identify or classify the different objects within the working environment, a performance risk may be associated with use of the robot. For example, the system might position itself within the working environment without colliding with any object, however, the robotic arm might define or follow a trajectory that points directly at a solid foreign object (mistakenly considering the solid foreign object as part of the patient's anatomy) such as an implant or a surgical tool. In such situations, the surgeon may be unable to access the desired target area for the surgery without first moving the solid foreign object or adjusting the trajectory.

Additionally, one or more anatomical features of a patient, and/or non-anatomical objects, in a volume of interest may move before or during a surgical procedure, but after an initial position thereof has been determined. In other words, a position of one or more objects within a volume of interest or work volume may change during a surgical procedure.

A need therefore exists for a system that can detect one or more structures or other objects within a robotic system's working environment. Such a system may be capable of identifying a precise position of the detected objects and/or of classifying the detected objects to prevent a collision risk and otherwise avoiding interrupting or harming execution of the clinical procedure and causing any harm to the patient. Such a system may also be configured to update a model of the robotic system's working environment (e.g., of a work volume or a volume of interest), based on information gathered from one or more sensors during a surgical procedure, and/or based on CAD models of one or more objects positioned within or planned to be positioned within the working environment. For example, where an implant tower is secured to a vertebra, one or more sensors (e.g., sensors used for segmental tracking of the spine or of spinal elements, an O-arm 2D long film scanner, sensors used for vertebrae/implants location detection, or any other sensors) may be used to detected a change in pose of the vertebra, and a CAD model of the implant tower may be utilized together with information about the detected change in pose of the vertebra to determine an updated position and/or orientation of the implant tower. This updated information may then be utilized to plan or modify one or more trajectories or other aspects of a surgical plan.

Embodiments of the present disclosure comprise a device or system that can detect and segment specific solid elements within a working environment, based on geometric properties, or a prior knowledge, or distinct features such as but not limited to reflecting infrared (IR) beads or geometric shapes. The device or system may receive a model of a portion of a patient's anatomy, which may comprise information about a planned and/or actual location of one or more foreign objects positioned within or proximate the patient's anatomy (e.g., within a volume of interest). The device or system may also update the model to include information about a planned or actual position of one or more objects within or proximate the patient's anatomy. The device or system may utilize this information for robotic motion control and/or to facilitate execution of a clinical procedure. For example, such information may be utilized to plan clinical implant insertion trajectories that will not collide with planned and/or detected objects in the volume of interest, and/or to modify clinical implant insertion trajectory to avoid a collision with an object that is planned to be or detected in the volume of interest, and/or to identify one or more objects in the path of a clinical implant insertion trajectory as well as a needed movement of the one or more objects to clear the trajectory.

Embodiments of the present disclosure may utilize one or more of information about a known robotic work volume, information (including position and/or orientation information) about one or more implants inserted by a robotic arm and/or with navigation system guidance, and/or registration information to extrapolate a location of objects that present interference risks with the work volume, and to update such location information based on the registration information or other information about an updated position of an anatomical feature, an implant, and/or any other object of interest. For example, if a screw with a screw tower has been inserted into a patient's vertebra, and the spine has later shifted (e.g., during insertion of an interbody implant), and another registration has been performed, then embodiments of the present disclosure may be configured to automatically update a model of the work volume based on, for example, known information about the tower (e.g., location information, size and/or shape information) and a difference in location of the tower from a first registration to the most recent registration. The updated model may then be used to plan and/or update a planned robotic movement, trajectory, or other procedure.

Embodiments of the present disclosure may also beneficially be used to detect a region of interest for a procedure and determine an alignment or other orientation of one or more anatomical elements within the region of interest. Such embodiments may utilize a visible marker placed on a patient or on a covering of the patient (in either case, whether resting thereon or secured thereto, with adhesive or otherwise), which marker may then be detected by a camera and/or an associated processor. A marker detection algorithm, distance information, and/or known information about the shape and size of the marker may be used to detect the marker in an image obtained by the camera. In one such embodiment, for example, where a surgical procedure is to be carried out on the spine of a patient with scoliosis (e.g., where the spine is pathologically curved), a visible marker may be positioned on a patient in alignment with anatomical feature of interest (e.g., a spine), which marker may then be detected using a camera. An orientation of the marker may be determined and then utilized to determine the angle of the spine section on which the operation is to take place, which determined angle may then be used to properly orient one or more surgical tools, implants, and/or other medical devices, and/or to adjust a surgical plan, and/or for other useful purposes. In some embodiments, the determined position or orientation may be used to position and align fiduciary markers utilized by a robot for registration purposes and/or to properly align or otherwise position the robot relative to the anatomical feature of interest.

Embodiments of the present disclosure may beneficially reduce or eliminate a need to utilize a navigation probe and/or to receive user input to determine an alignment or other orientation of the anatomical element(s) in question.

Embodiments of the present disclosure may utilize one or more mechanical or electronic inputs to scan/record/image a surgical working environment. Devices such as cameras (depth, infra-red, optical), proximity sensors, Doppler devices, and/or lasers may be utilized for this purpose. These devices may be located in the surgical environment, in a position such that it would detect the surgical working field. For examples, such devices may be mounted on a robotic arm, facing the surgical field, attached to a positioned navigation camera, or attached to an operating room lighting source.

Embodiments of the present disclosure may be utilized, for example, in connection with the use of a robot to operate a cutting tool to remove bone or other tissue from a patient, to reduce a likelihood that the robot will inadvertently remove or otherwise damage any anatomical feature or foreign object other than the targeted tissue.

The present disclosure provides a technical solution to the problems of (1) safely operating a robot in a surgical environment that comprises one or more objects that may be easily harmed or damaged and/or that are not intended to be modified or otherwise affected by the surgical procedure; (2) safely operating a robot in a crowded surgical environment; (3) safely operating a robot in a surgical environment with one or more objects that are moving during the course of a surgical procedure; (4) completing set-up of a robotic system for use in a surgical procedure as efficiently as possible; and/or (5) verifying that one or more objects identified in a surgical plan are in fact in a surgical environment, and/or determining an actual location of one or more objects identified in a surgical plan.

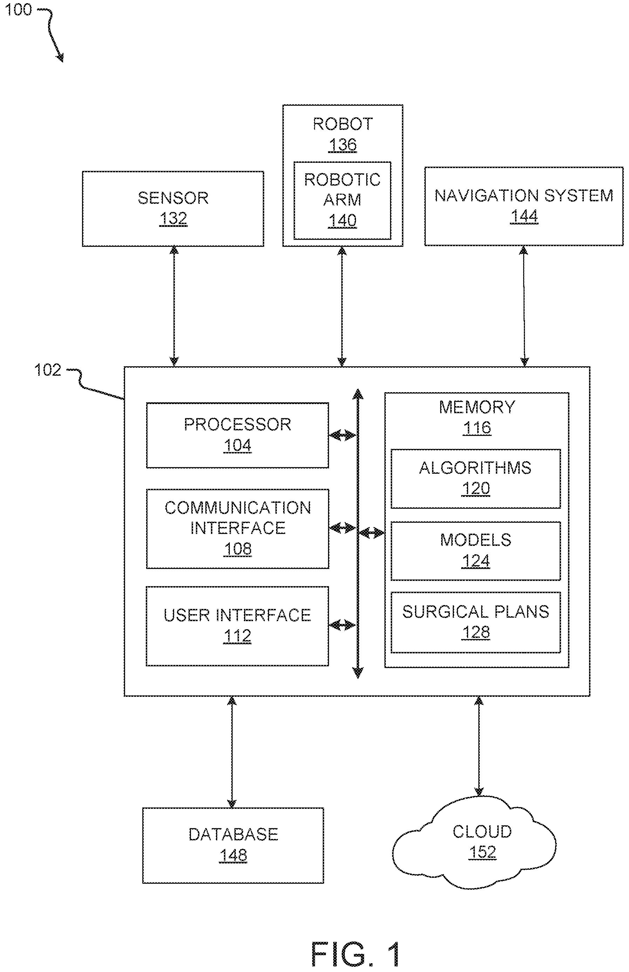

Turning first to Fig. 1, a block diagram of a system 100 according to at least one embodiment of the present disclosure is shown. The system 100 may be used, for example, to carry out a surgical procedure, to detect objects in a volume of interest, to identify such objects, to plan a path or trajectory for a robotic arm based on one or more detected objects, to determine a needed movement of one or more such objects to clear a path or trajectory, to update a surgical plan based on one or more detected and/or identified objects, to execute a surgical plan, to carry out one or more steps or other aspects of one or more of the methods disclosed herein, and/or for any other useful purpose. The system 100 comprises a computing device 102, one or more sensors 132, a robot 136, a navigation system 144, a database 148, and a cloud 152. Notwithstanding the foregoing, systems according to other embodiments of the present disclosure may omit any one or more of the one or more sensors 132, the robot 136, the navigation system 144, the database 148, and/or the cloud 152.

The computing device 102 comprises a processor 104, a communication interface 108, a user interface 112, and a memory 116. A computing device according to other embodiments of the present disclosure may omit one or both of the communication interface 108 and the user interface 112.

The processor 104 of the computing device 102 may be any processor described herein or any similar processor. The processor 104 may be configured to execute instructions stored in the memory 116, which instructions may cause the processor 104 to carry out one or more computing steps utilizing or based on data received, for example, from the sensor 132, the robot 136, the navigation system 144, the database 148, and/or the cloud 152.

The computing device 102 may also comprise a communication interface 108. The communication interface 108 may be used for receiving image or other data or other information from an external source (such as the sensor 132, the robot 136, the navigation system 144, the database 148, the cloud 152, and/or a portable storage medium (e.g., a USB drive, a DVD, a CD)), and/or for transmitting instructions, images, or other information to an external system or device (e.g., another computing device 102, the sensor 132, the robot 136, the navigation system 144, the database 148, the cloud 152, and/or a portable storage medium (e.g., a USB drive, a DVD, a CD)). The communication interface 108 may comprise one or more wired interfaces (e.g., a USB port, an ethernet port, a Firewire port) and/or one or more wireless interfaces (configured, for example, to transmit information via one or more wireless communication protocols such as 802.11a/b/g/n, Bluetooth, NFC, ZigBee, RF, GSM, LTE, and so forth). In some embodiments, the communication interface 108 may be useful for enabling the device 102 to communicate with one or more other processors 104 or computing devices 102, whether to reduce the time needed to accomplish a computing-intensive task or for any other reason.

The user interface 112 may be or comprise a keyboard, mouse, trackball, monitor, television, touchscreen, button, joystick, switch, lever, and/or any other device for receiving information from a user and/or for providing information to a user of the computing device 102. The user interface 112 may be used, for example, to receive a user selection or other user input in connection with any step of any method described herein; to receive a user selection or other user input regarding one or more configurable settings of the computing device 102 and/or of another component of the system 100; to receive a user selection or other user input regarding a desired movement of the robot 136; and/or to receive a user selection or other user input regarding a surgical objective; to receive a user selection or other user in put regarding a modification to a surgical plan. Notwithstanding the inclusion of the user interface 112 in the system 100, the system 100 may automatically (e.g., without any input via the user interface 112 or otherwise) carry out one or more, or all, of the steps of any method described herein.

Although the user interface 112 is shown as part of the computing device 102, in some embodiments, the computing device 102 may utilize a user interface 112 that is housed separately from one or more remaining components of the computing device 102. In some embodiments, the user interface 112 may be located proximate one or more other components of the system 100, while in other embodiments, the user interface 112 may be located remotely from one or more components of the system 100.

The memory 116 may be or comprise a hard drive, RAM, DRAM, SDRAM, other solid-state memory, any memory described herein, or any other tangible non-transitory memory for storing computer-readable data and/or instructions. The memory 116 may store instructions, information or data useful for completing, for example, any step of the methods 200, 300, 400, 500, 600, 650, 700, and/or 800 described herein. The memory 116 may store, for example, one or more algorithms 120 (including, for example, a marker detection algorithm, a feature recognition algorithm, an image processing algorithm, a trajectory calculation algorithm), one or more models 124 (including, for example, one or more CAD models of one or more implants, surgical tools, other medical devices, or any other models that provide, for example, information about the dimensions of an object, a shape of an object, material characteristics of an object, and/or mechanical properties of an object), and/or one or more surgical plans 128 (each of which may be or comprise, for example, one or more models or other three-dimensional images of a portion of an anatomy of a patient). Such instructions, algorithms, criteria, and/or templates may, in some embodiments, be organized into one or more applications, modules, packages, layers, or engines, and may cause the processor 104 to manipulate data stored in the memory 116 and/or received from another component of the system 100.

The sensor 132 may be any sensor suitable for obtaining information about surgical environment and/or about one or more objects in a working volume or other volume of interest. The sensor 132 may be or comprise, for example, a camera (including a visible light/optical camera, an infrared camera, a depth camera, or any other type of camera); a proximity sensor; and Doppler device; one or more lasers; a LIDAR device (e.g., a light detection and ranging device, and/or a laser imaging, detection, and ranging device); a scanner, such as a CT scanner, a magnetic resonance imaging (MRI) scanner, or an optical coherence tomography (OCT) scanner; an O-arm (including, for example, an O-arm 2D long film scanner), C-arm, G-arm, or other device utilizing X-ray-based imaging (e.g., a fluoroscope or other X-ray machine); sensors used for segmental tracking of the spine or of spinal elements; sensors used for vertebrae/implants location detection; an ultrasound probe; or any other imaging device suitable for obtaining images of a work volume or volume of interest. The sensor 132 may be operable to image an anatomical feature of a patient, such as a spine or a portion of a spine of a patient, as well as one or more objects positioned within, proximate, or otherwise around the anatomical feature of the patient. The sensor 132 may be capable of taking a 2D image or a 3D image to yield image data. "Image data" as used herein refers to the data generated or captured by an imaging device, including in a machine-readable form, a graphical form, and in any other form. In some embodiments, the sensor 132 may be capable of taking a plurality of 2D images from a plurality of angles or points of view, and of generating a 3D image by combining or otherwise manipulating the plurality of 2D images. In some embodiments, the system 100 may operate without the use of the sensor 132.

The sensor 132 may be operable to image a work volume or volume of interest in real-time (e.g., to generate a video feed or live stream). In such embodiments, the sensor 132 may continuously provide updated images and/or updated image data to the computing device 102, which may continuously process the updated images and/or updated image data as described herein in connection with one or more of the methods 200, 300, 400, 500, 600, 650, 700, and/or 800. In some embodiments, the sensor 132 may comprise more than one sensor 132. For example, a first sensor 132 may provide one or more preoperative images of an anatomical feature of a patient (which may be used, for example, to generate a surgical plan showing the anatomical feature of the patient as well as one or more implants or other objects to be inserted into or otherwise positioned in or proximate the anatomical feature of the patient), and a second sensor 132 may provide one or more intraoperative images of a work volume or other volume of interest comprising the anatomical feature of the patient (and/or one or more other objects and/or anatomical features) during a surgical procedure. In other embodiments, the same imaging device may be used to provide both one or more preoperative images and one or more intraoperative images.

The sensor 132 may be configured to capture information at a single point in time (e.g., to capture a still image or snapshot at a point in time), or to capture information in real time (e.g., to capture video information and/or a live stream of sensed information). The sensor 132 may be located in or proximate a surgical environment, and positioned so as to be able to detect a surgical working field or volume of interest. The sensor 132 may be, for example, mounted on a robotic arm such as the robotic arm 140, attached to a navigation camera (e.g., of a navigation system 144), attached to an operating room light, or held by a boom device or other stand.

The robot 136 may be any surgical robot or surgical robotic system. The robot 136 may be or comprise, for example, the Mazor X^{™} Stealth Edition robotic guidance system. The robot 136 may comprise one or more robotic arms 140. In some embodiments, the robotic arm 140 may comprise a first robotic arm and a second robotic arm. In other embodiments, the robot 126 may comprise one robotic arm, two robotic arms, or more than two robotic arms. The robotic arm 140 may, in some embodiments, hold or otherwise support the sensor 132. The robotic arm 140 may, in some embodiments, assist with a surgical procedure (e.g., by holding a tool in a desired trajectory or pose and/or supporting the weight of a tool while a surgeon or other user operates the tool, or otherwise) and/or automatically carry out a surgical procedure. In some embodiments, the system 100 may operate without the use of the robot 136.

The navigation system 144 may provide navigation for a surgeon and/or a surgical robot during an operation. The navigation system 144 may be any now-known or future-developed navigation system, including, for example, the Medtronic StealthStation^{™} S8 surgical navigation system. The navigation system 144 may include a camera or other sensor(s) for tracking one or more reference markers, navigated trackers, or other objects within an operating room or other room where a surgical procedure takes place. In various embodiments, the navigation system 144 may be used to track a position of the sensor 132 (or, more particularly, of a navigated tracker attached, directly or indirectly, in fixed relation to the sensor 132), and/or of the robot 136 (or one or more robotic arms 140 of the robot 136), and/or of any other object in a surgical environment. The navigation system 144 may include a display for displaying one or more images from an external source (e.g., the computing device 102, sensor 132, or other source) or a video stream from the camera or other sensor of the navigation system 144. In some embodiments, the system 100 may operate without the use of the navigation system 144.

In some embodiments, one or more reference markers (i.e., navigation markers) may be placed on the robot 136, the robotic arm 140, the sensor 132, or any other object in the surgical space. The reference markers may be tracked by the navigation system 144, and the results of the tracking may be used by the robot 136 and/or by an operator of the system 100 or any component thereof. In some embodiments, the navigation system 144 can be used to track other components of the system 100 (e.g., the sensor 132) and the system can operate without the use of the robot 136 (e.g., with a surgeon manually manipulating, based on guidance from the navigation system 144, any object useful for carrying out a surgical procedure).

The database 148 may store one or more images taken by one or more sensors 132 and may be configured to provide one or more such images (e.g., electronically, in the form of image data) to the computing device 102 (e.g., for display on or via a user interface 112, or for use by the processor 104 in connection with any method described herein) or to any other device, whether directly or via the cloud 152. In some embodiments, the database 148 may be or comprise part of a hospital image storage system, such as a picture archiving and communication system (PACS), a health information system (HIS), and/or another system for collecting, storing, managing, and/or transmitting electronic medical records including image data. The database 148 may store any of the same information stored in the memory 116 and/or any similar information. In some embodiments, the database 148 may contain a backup or archival copy of information stored in the memory 116.

The cloud 152 may be or represent the Internet or any other wide area network. The computing device 102 may be connected to the cloud 152 via the communication interface 108, using a wired connection, a wireless connection, or both. In some embodiments, the computing device 102 may communicate with the database 148 and/or an external device (e.g., a computing device) via the cloud 152.

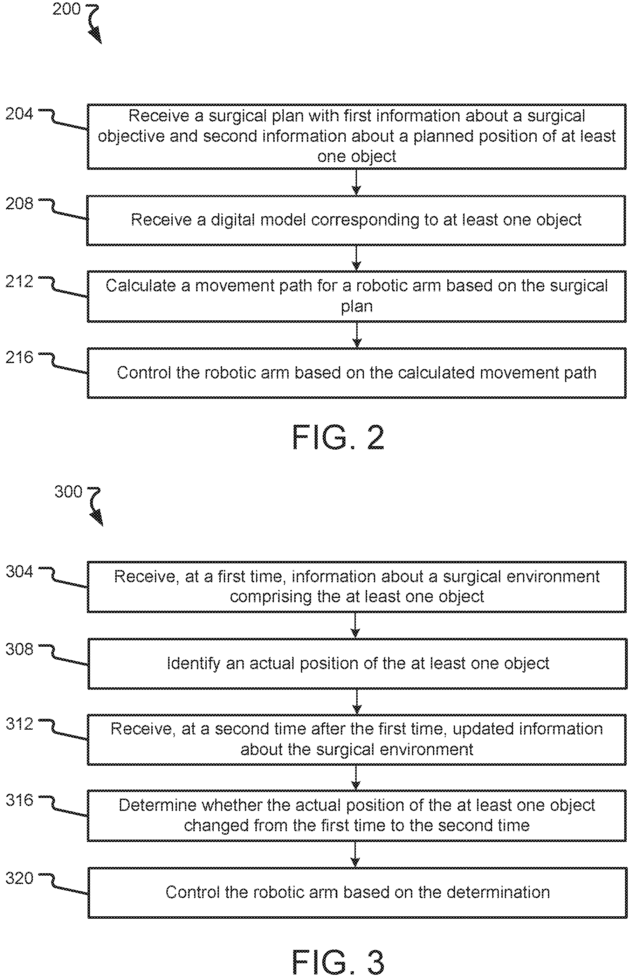

Turning now to Fig. 2, a method 200 for controlling a robotic arm may be utilized to reduce or eliminate a likelihood of a robotic arm of a robot colliding or otherwise interfering with an object positioned in a work volume of the robot, or vice versa. The method 200 beneficially improves patient safety, reduces the time required to complete a surgical procedure (by recognizing and planning for potential conflicts that might otherwise take extra time to resolve), and provides for the efficient completion of a surgical procedure. The method 200 may be carried out, for example, by a processor (e.g., the processor 104) executing instructions stored in a memory (e.g., the memory 116).

The method 200 comprises receiving a surgical plan with first information about a surgical objective and second information about a planned position of at least one object (step 204). The surgical plan may be a surgical plan 128, and may be received from the memory 116, or from the database 148, or from any other source. The surgical objective may be or comprise, for example, insertion of an implant into a particular location within an anatomy of a patient; removal of a certain portion of bony tissue from the patient's anatomy; removal of a certain portion of soft tissue from the patient's anatomy; correction of an anatomical defect in the patient's anatomy; removal of an anatomical feature from the patient; or any other surgical objective. In some embodiments, the surgical objective may comprise a main objective and one or more secondary objectives, or a plurality of unrelated objectives, or a plurality of objectives that, when completed in sequence, enable a subsequent objective until a main or primary objective is reached.

The at least one object may be or include any object planned to be used in connection with the surgical plan, including an implant, a surgical tool, or other medical device. In some embodiments, the at least one object may be a surgeon's, physician's, or other attending person's arm, hand, and/or finger(s) (e.g., for a surgical procedure to be completed manually with robotic assistance). The planned position may be a single planned position (e.g., a final position of an implant), or the planned position may comprise a plurality of positions corresponding to different positions of the at least one object at different stages of the surgical procedure.

The planned position of the at least one object may be defined in relation to a position of one or more anatomical elements, or in relation to a coordinate system. The coordinate system, in turn, may be defined with reference to one or more anatomical elements, or with reference to a reference marker, or with reference to any other object. The planned position may comprise a planned orientation as well (e.g., may be a planned pose).

The method 200 also comprises receiving a digital model corresponding to at least one object (step 208). The digital model may be a model 124 stored in the memory 116 or received from the database 148 via the communication interface 108 or otherwise. The digital model may, alternatively, be received from any other source. The digital model may comprise a CAD model of the at least one object, and may be useful for determining an exact size and/or shape of the medical implant. For example, where the at least one object is a medical implant, the digital model may comprise a CAD model of the medical implant. In some embodiments, the second information in the surgical plan may comprise information about a connection point of the at least one object to an anatomical feature of the patient, or other limited information about the at least one object, but may not comprise complete information about a size and/or shape of the at least one object. The digital model may be used, therefore, to supplement the second information so as to determine precisely which portion of a working volume of the robotic arm will be filled by the at least one object.

The method 200 also comprises calculating a movement path for a robotic arm based on the surgical plan (step 212). The robotic arm may be a robotic arm 140 of a robot 136. The movement path may be a path that enables the robotic arm to accomplish the surgical objective, or to accomplish a prerequisite step to accomplishing the surgical objective. The movement path may be, in some embodiments, not a path along which to move the robotic arm, but a trajectory to be defined by the robotic arm (together with, for example, an end effector or a surgical tool held by the robotic arm) and along which a surgical instrument will be moved to accomplish the surgical objective, or to accomplish a prerequisite step to accomplishing the surgical objective.

The movement path is calculated based on the surgical plan, and based more specifically on both the first information about the surgical objective and the second information about the planned position of the at least one object. The movement path is calculated to avoid interference between the robotic arm (or a surgical instrument or tool that will be held and/or guided by the robotic arm) and the at least one object, and also to ensure that the surgical objective is met (or that progress toward meeting the surgical objective is achieved). In some embodiments, the movement path may be calculated based also on the digital model. Thus, for example, the calculating may comprise determining a precise position of the at least one object based on the surgical plan and the digital model, and then calculating a movement path that avoids any interference (including any collision) between the at least one object and the robotic arm (or an end effector of or tool held by the robotic arm).

The movement path may be calculated using, for example, one or more algorithms such as the algorithms 120.

The method 200 also comprises controlling the robotic arm based on the calculated movement path (step 216). For example, the robotic arm may be caused to move along the movement path, or the robotic arm may be caused to position a tool guide so as to define a trajectory along the movement path, or the robotic arm may be caused to move a surgical tool along the movement path. The controlling may comprise generating one or more command signals and transmitting the one or more command signals to the robot 136, whether via a communication interface such as the communication interface 108 or otherwise.

The present disclosure encompasses embodiments of the method 200 that comprise more or fewer steps than those described above, and/or that comprise executing the steps described above in a different order than described above. The present disclosure also encompasses embodiments of the method 200 that comprise one or more steps other than those described above, including any one or more steps of any of the methods 300, 400, 500, 600, 650, 700, and/or 800 described herein.

Turning now to Fig. 3, a method 300 for determining whether a position of an object within a volume of interest has changed may beneficially be used to reduce a likelihood of a robot or a portion thereof (e.g., a robotic arm) from colliding with an object because the object has moved out of a previously known location. One or more aspects of the method 300 may be utilized for other purposes, such as to identify an initial position of an object within a surgical environment. The method 300 beneficially improves patient safety, reduces the time required to complete a surgical procedure (by recognizing and planning for potential conflicts that might otherwise take extra time to resolve), and provides for the efficient completion of a surgical procedure. The method 300 may be carried out, for example, by a processor (e.g., the processor 104) executing instructions stored in a memory (e.g., the memory 116).

The method 300 comprises receiving, at a first time, information about a surgical environment comprising at least one object (step 304). The information may be received from a sensor such as the sensor 132, and may be or comprise image data. The information may be or comprise a digital model that includes or is based on image data. The information may alternatively comprise information provided by a user of a system such as the system 100, and may be input via a user interface such as a user interface 112. The first time may be a time before a surgical procedure has begun (whether immediately before, or during or in connection with a preoperative consultation), a time during execution of a surgical procedure, a time when the patient is in an operating room, a time when the patient is not in the operating room, or another time.

The method 300 also comprises identifying an actual position of the at least one object (step 308). The identifying may be based on the information about the surgical environment received during the step 304, and may comprise applying a feature detection algorithm, feature recognition algorithm, edge detection algorithm, or other algorithm 120. The identifying may additionally or alternatively be based on navigation information and/or registration information that correlates the information about the surgical environment to a coordinate system such as a patient coordinate system, a robotic coordinate system, a global coordinate system, or any other coordinate system. The identifying may, in some embodiments, comprise receiving a digital model (e.g., a CAD model or other model 124) of the at least one object, and either analyzing the information based at least in part on the digital model or supplementing the information with the digital model. The actual position of the at least one object may be stored in a memory such as the memory 116 or database 148, and may in some embodiments be used to plan a trajectory or movement path for a surgical procedure.

In some embodiments, the identifying may be based at least in part on a surgical plan that identifies an expected or predicted position of the at least one object. The identifying may further comprise comparing the actual position of the at least one object to the expected or predicted position of the at least one object according to a surgical plan, and/or updating the surgical plan with and/or based on the actual position of the at least one object.

The method 300 also comprises receiving, at a second time after the first time, updated information about the surgical environment (step 312). The receiving at the second time may be the same as or substantially similar to the receiving at the first time of the step 304. The updated information may be information from the same sensor or other source that from which the information was received at the first time, or the updated information may be received from a different sensor or other source. In some embodiments, the updated information may be real-time information or may be selected or extracted from real-time information.

The method 300 also comprises determining whether the actual position of the at least one object changed from the first time to the second time (step 316). The step 316 may comprise repeating the step 308 (or a variation of the step 308) based on the updated information. In other words, the step 316 may comprise determining an actual position of the at least one object based on the updated information, whether in the same manner as or in a similar manner to the identification of the actual position of the at least one object in the step 308.

The determining comprises comparing a position of the at least one object corresponding to the updated information to the actual position of the at least one object as determined in the step 308. In some embodiments, the comparing may comprise overlaying the updated information on the information received in the step 304, determining whether any points in the updated information are different than any corresponding points in the information, and if so determining whether such points correspond to the at least one object.

In other embodiments, the determining may comprise determining an actual position of the at least one object based on the updated information (whether in the same manner as described above with respect to the step 304, a similar manner, or a different manner), and then comparing the actual position of the at least one object in the updated information to the actual position of the at least one object as determined in the step 308.

In some embodiments, a surgical plan may be updated based on the results of the determination. For example, if the actual position of the at least one object has changed, the surgical plan may be updated to reflect the updated actual position of the at least one object. If the at least one object is or includes an obstacle to a surgical procedure, and/or if the at least one object is or includes a target of a surgical procedure, then one or more trajectories or movement paths of a robotic arm, surgical tool, or other device may be calculated and/or updated based on updated actual position of the at least one object (e.g., to avoid collisions between the robotic arm, surgical tool, or other device and the at least one object, and/or to ensure that the robotic arm, surgical tool, or other device is properly directed to the target).

Where the at least one object is secured to or otherwise has a fixed position relative to another object (e.g., where the at least one object is a pedicle screw fixed in a vertebra), the determining may comprise determining an updated position of the object to which the at least one object is secured.

In embodiments where the first time is after an initial registration procedure (during which, for example, a coordinate space of a patient is correlated to a coordinate space of a surgical robot and/or of a navigation system), the determining whether the actual position of the at least one object has changed from the first time to the second time may comprise determining that re-registration is necessary due to movement of the at least one object.

Also in some embodiments, the step 316 may be replaced by a simple determination of an updated actual position of the at least one object, without reference to any previous determination of the actual position of the at least one object.

The method 300 also comprises controlling the robotic arm based on the determination (step 320). The controlling may comprise moving the robotic arm along an updated movement path generated based on the determination in the step 316, and/or based on an updated actual position of the at least one object. The controlling may comprise moving the robotic arm to position a tool guide or other instrument along a trajectory generated based on the determination in the step 316, and/or based on an updated actual position of the at least one object. The controlling may additionally or alternatively comprise stopping movement of the robotic arm upon determining that a position of the at least one object has changed. The controlling may be the same as or similar to the step 216 described above.

The present disclosure encompasses embodiments of the method 300 that comprise more or fewer steps than those described above, and/or that comprise executing the steps described above in a different order than described above. The present disclosure also encompasses embodiments of the method 300 that comprise one or more steps other than those described above, including any one or more steps of any of the methods 200, 400, 500, 600, 650, 700, and/or 800 described herein.

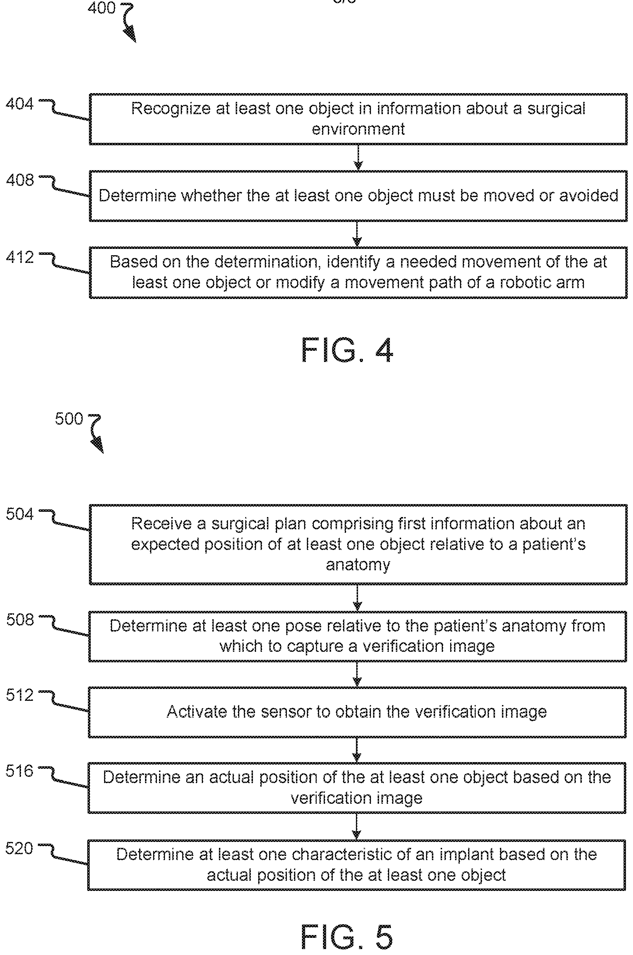

Fig. 4 describes a method 400 for reducing or eliminating a likelihood of a robotic arm of a robot colliding or otherwise interfering with an object positioned in a work volume of the robot, or vice versa. The method 400 beneficially improves patient safety, reduces the time required to complete a surgical procedure (by recognizing and planning for potential conflicts that might otherwise take extra time to resolve), and provides for the efficient completion of a surgical procedure. The method 400 may be carried out, for example, by a processor (e.g., the processor 104) executing instructions stored in a memory (e.g., the memory 116).

The method 400 comprises recognizing at least one object in information about a surgical environment (step 404). The recognizing may comprise receiving the information about the surgical environment, whether via a communication interface such as the communication interface 108 or otherwise. The recognizing may additionally or alternatively comprise applying a feature detection algorithm, feature recognition algorithm, edge detection algorithm, or other algorithm 120 to the information to detect the at least one object. The recognizing may further comprise sending and/or receiving information to and/or from, respectively, a database comprising information about one or more objects, to facilitate recognition of the at least one object. For example, the recognizing may comprise accessing a look-up table using one or more characteristics about an item detected in the information, and based on the characteristics identifying to which of a plurality of objects described in the look-up table the item corresponds. In some embodiments, the recognizing may comprise referring to a surgical plan to determine which object(s) are expected to be in a surgical environment as well as, in some embodiments, a planned or expected position of the object(s). Thus, for example, if at least one object detected in the information about the surgical environment is positioned in the same information as an implant identified in the surgical plan, then the recognizing may comprise comparing one or more characteristics of the detected object to one or more corresponding characteristics of the object(s) in the surgical plan to determine whether they are the same object(s).

In some embodiments, the recognizing may comprise referencing an anatomical atlas to determine whether the at least one object is an anatomical feature. Where the object is an anatomical feature, one or more algorithms or some other logic may be used to determine whether the anatomical feature is an anatomical feature of a patient (e.g., an organ, artery, nerve, bone, or other anatomical feature within the surgical environment) or of a surgeon or other operating room personnel (e.g., an arm, hand, and/or finger of a surgeon, positioned in the surgical environment to assist with a surgical procedure).

In some embodiments, the at least one object may be an incision through which some or all of a surgical procedure will be performed, and through which one or more surgical instruments or tools must pass in order to carry out a surgical procedure. In such embodiments, the recognizing may comprise detecting or otherwise identifying one or more markers placed in or proximate the incision, and/or one or more tools being used to keep the incision open, and/or the incision itself.

The method 400 also comprises determining whether the at least one object must be moved or avoided (step 408). The determining may comprise, for example, determining whether the at least one object is a target (e.g., an anatomical feature to be modified, fixed, or removed, or a pedicle screw to which a vertebral rod is to be secured) or an obstacle (e.g., an anatomical feature that needs to be protected during a surgical procedure to avoid damage thereto; or a medical instrument or device such as a tube, catheter, retractor, implant, or other implement that must be protected or that presents a collision risk for a robotic arm or tool on a predetermined trajectory; or an arm, hand, and/or finger of a surgeon or other operating room personnel). The determining may also comprise evaluating whether an existing planned movement path or trajectory conflicts with the at least one object (e.g., if the at least one object is an obstacle) or intersects with or otherwise reaches the at least one object (e.g., if the at least one object is a target).

Where the at least one object is a target, the determining may comprise concluding that the at least one object should be neither moved nor avoided. Alternatively, the determining may comprise concluding that the at least one object can be moved by the robotic arm or another surgical tool. Where the at least one object is, for example, an incision, the determining may comprise concluding that insertion by a robot of a surgical tool through the incision will enable a surgical objective to be accomplished even if the incision is not precisely in a planned location, whether because the robot will be able to achieve a planned movement path or trajectory by pushing against an edge or side of the incision (and thus "moving" the incision, at least relative to one or more internal features of the patient) or otherwise. Where the object is an obstacle, the determining may comprise evaluating whether the obstacle is movable (e.g., whether the obstacle may be moved by a robot or surgical tool or otherwise) or must simply be avoided (e.g., by modifying a planned movement path and/or trajectory). Where an object is movable but may also be avoided, then the determining may comprise evaluating whether to move or avoid the object based one or more predetermined criteria, which may be or include the amount of time required for each option; which option is least likely to cause trauma to the patient; which option is most likely to yield a positive surgical outcome, and/or any other criterion.

The method 400 also comprises identifying a needed movement of the at least one object or modifying a movement path of a robotic arm, in each case based on the determination (step 412). For example, where the at least one object is determined to be an obstacle that can be moved, then a needed movement of the at least one object from a current position to a new position may be identified so as to clear a movement path or trajectory for or associated with a robotic arm or other surgical tool. Where the at least one object is determined to be an obstacle that cannot be moved, then a movement path or planned trajectory of or associated with a robotic arm may be modified to avoid a potential collision with the at least one object. Where the at least one object is determined to be a target that can be moved, then a needed movement of the at least one object may be determined to ensure that the at least one object may be successfully reached (e.g., using a predetermined movement path or trajectory). Where the at least one object is determined to be a target that cannot be moved, then a movement path or trajectory of or associated with a robotic arm may be modified to ensure that that the at least one object may be successfully reached.

In some embodiments, the identifying or modifying may be based at least in part on a surgical plan that identifies an expected or predicted position of the at least one object. The identifying or modifying may further comprise comparing the actual position of the at least one object to the expected or predicted position of the at least one object according to a surgical plan, and/or updating the surgical plan with and/or based on the actual position of the at least one object.

The present disclosure encompasses embodiments of the method 400 that comprise more or fewer steps than those described above, and/or that comprise executing the steps described above in a different order than described above. The present disclosure also encompasses embodiments of the method 400 that comprise one or more steps other than those described above, including any one or more steps of any of the methods 200, 300, 500, 600, 650, 700, and/or 800 described herein.

Fig. 5 describes a method 500 for verifying a position of an object relative to a patient's anatomy. The method 500 beneficially improves patient safety, reduces the time required to complete a surgical procedure (by recognizing and planning for potential conflicts that might otherwise take extra time to resolve), and provides for the efficient completion of a surgical procedure. The method 500 may be carried out, for example, by a processor (e.g., the processor 104) executing instructions stored in a memory (e.g., the memory 116).

The method 500 comprises receiving a surgical plan comprising first information about an expected position of at least one object (step 504). The surgical plan may be any preoperative plan comprising information about one or more planned steps of a surgical procedure and/or about one or more features of a patient's anatomy. The surgical plan may be the same as or similar to, for example, a surgical plan 128. The surgical plan may be received from a memory such as the memory 116, a database such as the database 148, or any other source. The surgical plan may be received via a communication interface such as the communication interface 108, and/or a cloud or other network such as the cloud 152. In some embodiments, the surgical plan may be received via a user interface such as the user interface 112.

The at least one object may be an implant, a surgical tool or instrument, or any other medical device. For example, the at least one object may be a vertebral screw or screw tower. The at least one object may be a particular anatomical feature of the patient, such as a vertebra or other bone, or an organ, or a tumor. The at least one object may be an object that exists within the patient independent of a surgical procedure corresponding to the surgical plan, or an object that will be inserted into the patient or used in connection with the surgical procedure corresponding to the surgical plan.

The expected position is relative to the anatomy of a patient, and may be based on one or more preoperative images of the patient, which images may comprise information about the at least one object and/or about the patient's anatomy proximate the at least one object. For example, where the at least one object is a vertebra, one or more preoperative images may be used to determine an expected position of the vertebra relative to one or more other vertebra and/or one or more other anatomical features of the patient. The expected position may be a planned position of a surgical tool or instrument, which may in turn correspond to a particular step of a planned surgical procedure. For example, where the at least one object is a retractor, the expected position may be a planned position of the retractor based on a planned incision location. Where the at least one object is an implant, the expected position may be a position into which the implant is planned to be inserted to accomplish a medical objective.

The first information about the expected position of the at least one object may be, for example, a set of coordinates in a coordinate system. The coordinate system may be a patient coordinate system, or a robotic coordinate system, or a navigation coordinate system, or any other coordinate system. The first information may be or comprise relative position information, such as one or more distances from one or more points (e.g., one or more unique anatomical points). The first information may be or comprise information from which an expected position of the at least one object may be determined or calculated.

The method 500 also comprises determining at least one pose from which to capture a verification image (step 508). The at least one pose may be a pose of an imaging device with which the verification image will be captured, and may additionally or alternatively be or correspond to a pose of a robotic arm configured to hold such an imaging device. The at least one pose may be determined using one or more algorithms such as the algorithms 120, and/or using one or more models such as the models 124. The at least one pose may be determined to ensure that the at least one object as well as one or more suitable reference points are within the verification image. The at least one pose may additionally or alternatively be determined to ensure that an imaging device with which the verification image will be taken has a clear line of sight to the at least one object. The at least one pose may be determined to ensure that a position and/or orientation of the at least one object can be determined.

The at least one pose may also be determined based on one or more characteristics of an imaging device with which the verification image will be obtained. For example, where the imaging device is an ultrasound probe, the at least one pose may be a pose proximate a surface of the patient's skin and may be determined to ensure that the at least one object is not, for example, in the shadow of a bone. Where the imaging device is an optical coherence tomography (OCT) camera, the at least one pose may again be a pose proximate a surface of the patient's skin. Where the imaging device is an X-ray imaging device, the at least one pose may be determined to ensure that the at least one object will be properly imaged by the X-ray imaging device, and (in some embodiments) to avoid unnecessary exposure to radiation by portions of the patient's anatomy that are not of interest.

The method 500 also comprises activating a sensor to obtain the verification image (step 512). The sensor may be the same as or similar to the sensor 132. The activating may comprise transmitting a command to the sensor that causes the sensor to obtain the verification image, or otherwise causing the sensor to obtain the verification image. In some embodiments, the step 512 may comprise receiving a verification image obtained or captured using a sensor rather than activating a sensor to obtain the verification image. The verification image may be a single verification image or a plurality of verification images. For example, where the step 508 yields a determination of multiple poses, the step 512 may comprise activating the sensor to obtain a verification image at each of the determined poses, or receiving a verification image captured or taken by the sensor in each of the determined poses.

The method 500 also comprises determining an actual position of the at least one object based on the verification image (step 516). The determining may comprise utilizing one or more algorithms (such as, for example, the algorithms 120) to detect the at least one object in the verification image, to detect one or more features in the verification image other than the at least one object (e.g., one or more anatomical features, reference markers or points, or other features), and to determine a position of the at least one object relative to one or more other features in the verification image. The determining may also comprise utilizing information about the at least one pose from which the verification image was taken to determine an actual position of the at least one object. The actual position of the at least one object may be or comprise any position information regarding the at least one object as determined using the verification image and/or other information about a current state of the at least one object, as opposed to information regarding a planned position of the at least one object (e.g., from the surgical plan).

The method 500 also comprises adjusting the surgical plan based on the actual position of the at least one object (step 520). The adjusting may comprise, for example, moving a representation of the at least one object in the surgical plan from a planned position (e.g., relative to one or more features of the patient's anatomy or some other reference) to the actual position. The adjusting may comprise updating an incision location, a trajectory, a tool to be used, a position of a navigation camera or other sensor, a position of a robot, a planned pose of a robotic arm, or any other feature of the surgical plan. Where the at least one object is a screw tower or a group of screw towers, for example, the adjusting may comprise determining a needed contour of a vertebral rod based on the actual position of the screw tower or group of screw towers, so that the vertebral rod will pass through each of the screw towers and can be secured to the corresponding screws.

The present disclosure encompasses embodiments of the method 500 that comprise more or fewer steps than those described above, and/or that comprise executing the steps described above in a different order than described above. The present disclosure also encompasses embodiments of the method 500 that comprise one or more steps other than those described above, including any one or more steps of any of the methods 200, 300, 400, 600, 650, 700, and/or 800 described herein.

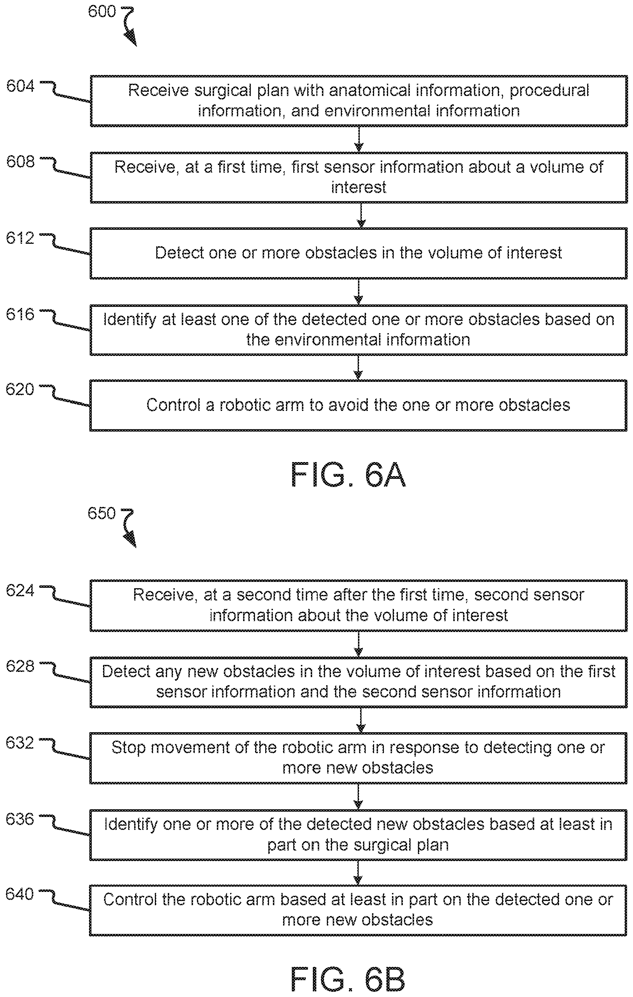

Fig. 6A describes a method 600 for controlling a robotic arm so as to avoid one or more obstacles. The method 600 beneficially improves patient safety, reduces the time required to complete a surgical procedure (by recognizing and planning for potential conflicts that might otherwise take extra time to resolve), and provides for the efficient completion of a surgical procedure. The method 600 may be carried out, for example, by a processor (e.g., the processor 104) executing instructions stored in a memory (e.g., the memory 116).

The method 600 comprises receiving a surgical plan with anatomical information, surgical information, and environmental information (step 604). The surgical plan may be, for example, a surgical plan 128 stored in a memory such as the memory 116. The anatomical information may be or comprise, for example, information about an anatomical portion of a patient, and may include information about the position of one or more anatomical features, whether relative to one or more other anatomical features (as in an image, for example), one or more other objects, or one or more coordinate systems. The anatomical information may comprise information about one or more characteristics of one or more anatomical features as well. The surgical information may be or comprise, for example, information about one or more planned surgical procedures, and may include information about a planned trajectory for inserting one or more implants, a planned position and/or orientation of one or more implants, one or more tools or other instruments planned to be used during the surgical procedure, a planned position and orientation of an incision, instructions for carrying out one or more steps of a surgical procedure, and/or any similar or related information. The environmental information may comprise information about one or more objects that are expected to be positioned proximate to or within the patient (e.g., within the surgical environment) during a surgical procedure. The environmental information may comprise information about one or more medical devices, implants, surgical tools, or other manmade objects. The environmental information may also comprise information about one or more planned movements of a surgeon or other operating room staff relative to the patient. For example, the environmental information may comprise information about an expected position of an arm, hand, or finger of a surgeon or other operating room staff at one or more points in time during a surgical procedure.

The method 600 also comprises receiving, at a first time, first sensor information about a volume of interest (step 608). The sensor information may be obtained from a sensor such as the sensor 132, and may comprise an image or other image data, or non-image data, about the volume of interest. The volume of interest may be or correspond to the anatomical portion of the patient to which the anatomical information in the surgical plan relates. In some embodiments, the volume of interest may be larger or smaller than the anatomical portion of the patient to which the anatomical information in the surgical plan relates.

The method 600 also comprises detecting one or more obstacles in the volume of interest (step 612). The one or more obstacles may be or include one or more objects referenced in the environmental information of the surgical plan. The detecting may comprise utilizing a feature recognition algorithm, an edge detection algorithm, an algorithm generated using machine learning, or any other algorithm (including any algorithm 120) to detect the one or more obstacles in the volume of interest. The obstacles may be anatomical features of a patient that lie along a trajectory identified in the surgical plan, or any other object that is or may be in the way of a robotic arm used to assist with or to carry out a surgical procedure described in the surgical plan. For example, obstacles may be or include implants, surgical tools or other medical instruments, tubes, and/or anatomical features (e.g., hands, fingers, arms) of a surgeon or other operating room staff. The detecting may comprise comparing the first sensor information to information in the surgical plan (e.g., anatomical information, environmental information) to identify one or more objects or other items that are represented in the first sensor information but not in the surgical plan. The detecting may also comprise comparing the first sensor information to information in the surgical plan to identify one or more objects or other items that are expected to be in the volume of interest, but that nevertheless comprise or constitute an obstacle or potential obstacle to movement of the robotic arm.

The method 600 also comprises identifying at least one of the detected one or more obstacles based on the environmental information (step 616). The identifying may comprise correlating each of the one or more identified obstacles with an object described or otherwise represented in the environmental information. The correlating may be based on one or more of a position of the detected obstacle (e.g., relative to a planned or expected position of an object as reflected in the environmental information), a size of the detected obstacle (e.g., relative to a size of an object as reflected in the environmental information), a shape of the detected obstacle (e.g., relative to a shape of an object as reflected in the environmental information), and/or any other information about or characteristic of the detected obstacle and corresponding information about or characteristic of an object as reflected in the environmental information.

In some embodiments, the identifying may also comprise utilizing one or more digital models, such as the models 124. For example, where the environmental information comprises an indication that a certain implant or tool is planned to be used in connection with a surgical procedure, that information may be used to locate a model of the implant or tool, from which information about the implant or tool may be obtained (including, for example, information about the size, shape, and/or material composition of the implant or tool). The information so obtained from the model may then be used, whether standing alone or together with other information contained in the environmental information (such as, for example, information about an expected or planned position of the implant or tool within the volume of interest) to facilitate identification of the at least one of the one or more obstacles.

Identification of an obstacle may beneficially enable one or more additional determinations, such as whether the obstacle is fixed or can be moved (e.g., by application of an external force thereto); whether the obstacle is likely to move during the surgical procedure (whether due to movement of an anatomical feature or other object to which the obstacle is attached, or otherwise); and/or whether the obstacle is highly sensitive to damage or not (which information may be used to determine, for example, how far away from the obstacle the robotic arm will stay, and/or how quickly the robotic arm will move when proximate the obstacle). Identification of an obstacle may also beneficially help to avoid misidentification of an object as an obstacle (rather than as, for example, a target).

The method 600 also comprises controlling a robotic arm to avoid the one or more obstacles (step 620). The controlling may comprise generating a movement path based on a known location of the one or more obstacles, and then causing the robotic arm to move along the movement path. The controlling may also comprise stopping movement of the robotic arm if the robotic arm is determined to be unacceptably close to an obstacle, or causing the robotic arm to deviate from a current movement path in order to avoid colliding with or otherwise impacting an obstacle. The controlling may comprise sending one or more signals to a robot that comprises the robotic arm, which signals cause the robot to move the robotic arm in a particular way. The controlling may also comprise opening a power circuit through which the robot receives power, so as to immediately stop the robot. The controlling may further comprise selectively operating one or more motors in the robotic arm to cause the robotic arm to move along a desired path.

The present disclosure encompasses embodiments of the method 600 that comprise more or fewer steps than those described above, and/or that comprise executing the steps described above in a different order than described above. The present disclosure also encompasses embodiments of the method 600 that comprise one or more steps other than those described above, including any one or more steps of any of the methods 200, 300, 400, 500, 650, 700, and/or 800 described herein.

Fig. 6B describes a method 650 for detecting and avoiding new obstacles in a volume of interest. As described herein, the method 650 comprises additional steps that may be completed in connection with the method 600, although in some embodiments one or more steps of the method 650 may be completed independently of one or more steps of the method 600. The method 650 beneficially improves patient safety, reduces the time required to complete a surgical procedure (by recognizing and avoiding potential conflicts that might otherwise take extra time to resolve), and provides for the efficient completion of a surgical procedure. The method 650 may be carried out, for example, by a processor (e.g., the processor 104) executing instructions stored in a memory (e.g., the memory 116).

The method 650 comprises receiving, at a second time after the first time, second sensor information about the volume of interest (step 624). The receiving of the second sensor information may be the same as or substantially similar to the receiving of the first sensor information as described above in connection with the step 608, except that the second sensor information is received at a second time after the first time.

The method 650 also comprises detecting any new obstacles in the volume of interest based on the first sensor information and the second sensor information (step 628). The detecting may be completed in the same way, or in a substantially similar way, to the detecting of one or more obstacles in the volume of interest as described above in connection with the step 612 of the method 600. In some embodiments, the detecting may comprise comparing the second sensor information to the first sensor information; ignoring, deleting, or otherwise removing from consideration those portions of the second sensor information that are substantially identical to the first sensor information (e.g., portions of the second sensor information that are no different from the first sensor information; and then analyzing the remaining second sensor information to detect any new obstacles therein. Thus, for example, if the first sensor information and the second sensor information each comprise an image of a portion of a patient's spine, then the detecting may comprise overlaying the image from the second sensor information on the image from the first sensor information, removing from consideration every part of the image from the second sensor information that is the same as the image from the first sensor information, and then analyzing the remaining portions of the image to identify any new obstacles therein. The analyzing in particular may be completed in the same manner or in a substantially similar manner to the detecting one or more obstacles in the volume of interest as described above in connection with the step 612 of the method 600.

The method 650 also comprises stopping movement of the robotic arm in response to detecting one or more new obstacles (step 632). The stopping may comprise sending a signal to a robot comprising the robotic arm that causes the robot to stop movement of the robotic arm, or the stopping may comprise cutting power to the robot so that the robot is incapable of further movement. The stopping may occur only if the robotic arm is on a collision course with the one or more new obstacles, or the stopping may occur prior to or in the absence of a determination that the robotic arm is on a collision course with the one or more new obstacles. In some embodiments, the stopping may occur so that a determination as to whether the robotic arm is on a collision course with the one or more new obstacles can be made.

The method 650 also comprises identifying one or more of the detected new obstacles based at least in part on the surgical plan (step 636). The identifying may be the same as or similar to the identifying at least one of the detected one or more obstacles as described above in connection with the step 616 of the method 600.

The method 650 also comprises controlling the robotic arm based at least in part on the detected one or more new obstacles (step 640). The controlling may comprise generating a movement path based on a known location of the detected one or more new obstacles, and then causing the robotic arm to move along the movement path. The controlling may also comprise stopping movement of the robotic arm if the robotic arm is determined to be unacceptably close to one of the detected one or more new obstacles, or causing the robotic arm to deviate from a current movement path in order to avoid colliding with or otherwise impacting one of the detected one or more new obstacles. The controlling may comprise sending one or more signals to a robot that comprises the robotic arm, which signals cause the robot to move the robotic arm in a particular way. The controlling may also comprise opening a power circuit through which the robot receives power, so as to immediately stop the robot. The controlling may further comprise selectively operating one or more motors in the robotic arm to cause the robotic arm to move along a desired path.

The present disclosure encompasses embodiments of the method 650 that comprise more or fewer steps than those described above, and/or that comprise executing the steps described above in a different order than described above. The present disclosure also encompasses embodiments of the method 650 that comprise one or more steps other than those described above, including any one or more steps of any of the methods 200, 300, 400, 500, 600, 700, and/or 800 described herein.

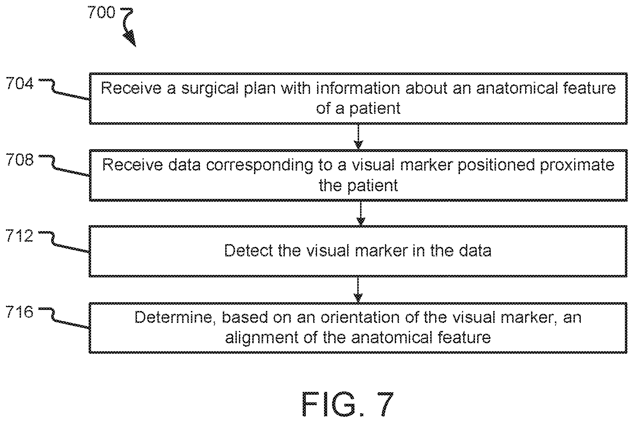

Fig. 7 describes a method 700 for determining an alignment of an anatomical feature of a patient. The method 700 beneficially reduces the amount of time needed to set up a surgical robot for use in a surgical procedure, as well as the cost associated with the surgical procedure. By eliminating one or more steps that may otherwise be required during setup for a surgical procedure, the method 700 also reduces the opportunity for errors to be made and aids in reducing surgeon fatigue, thus contributing to improved patient safety. The method 700 may be carried out, for example, by a processor (e.g., the processor 104) executing instructions stored in a memory (e.g., the memory 116).

The method 700 comprises receiving a surgical plan with information about an anatomical feature of a patient (step 704). The surgical plan may be, for example, a surgical plan 128. The anatomical feature may be any anatomical feature of a patient, including, for example, a spine (or portion thereof) of a patient, a joint of a patient, and/or a skull of a patient. The anatomical feature may be a feature whose alignment is visible upon visual examination of the patient (e.g., without the use of X-rays or other images, and without making any incisions or otherwise gaining access to the patient's interior).

The surgical plan may comprise information about a surgical procedure to be carried out that involves the anatomical feature of the patient. For example, the surgical plan may comprise information about a surgery to correct severe spinal scoliosis. The surgical plan may further comprise information useful by or in connection with a surgical robot for carrying out the surgical procedure or assisting with one or more aspects thereof.

The method 700 further comprises receiving data corresponding to a visual marker positioned proximate the patient (step 708). The visual marker may have or comprise one or more distinguishing characteristics to facilitate detection thereof, such as a unique or distinct or particular color, shape, contour, geometric patterns, light patterns, markers, or other indicia. The visual marker may have been placed (e.g., by a surgeon or other operating room staff) on a patient so as to be aligned with the anatomical feature of the patient. For example, where the anatomical feature is a spine, the visual marker may have been placed on the patient with a predetermined dimension or axis thereof (e.g., a length dimension, or a longitudinal axis) aligned with the spine. The visual marker may be resting on the patient, or may be adhered to the patient in some way (e.g., may comprise a sticker, or a non-slip/high friction pad that rests on the patient), or may be otherwise secured to the patient (e.g., may be tied to the patient, or taped to the patient), and may be easily removable from the patient. The visual marker may be placed directly on the patient's skin, or may be affixed to a towel, cloth, drape, or other covering that rests on top of the patient. The visual marker may have one or more markings thereon to indicate to a surgeon or other operating staff which dimension or axis thereof should be aligned with the anatomical feature.

Although described herein as a visual marker, in some embodiments of the present disclosure, a marker may be used in place of the visual marker that may be detected by a non-visual or non-optical sensor. For example, a marker comprising a unique magnetic signature may be used instead of a visual marker.

The received data may be, for example, an image of the visual marker on the patient or other image data corresponding to the visual marker, and may be received (whether directly or indirectly) from a camera or other sensor.

The method 700 also comprises detecting the visual marker in the data (step 712). The detecting may comprise utilizing a feature recognition algorithm, an edge detection algorithm, an algorithm generated using machine learning, or any other algorithm (including any algorithm 120) to detect the visual marker. The detecting may comprise searching the data for a particular color, shape, contour, geometric pattern, light pattern, marker, or other indicia (or any representation in the data of any of the foregoing) known to be associated with the visual marker.

The method 700 also comprises determining, based on an orientation of the visual marker, an alignment of the anatomical feature (step 716). The determining may comprise identifying an orientation of the visual marker based on the data, and assigning or otherwise correlating a corresponding orientation to the anatomical feature in question. For example, where the surgical plan corresponds to a spinal surgical procedure, and the visual marker has been placed on the patient so as to align with the portion of the patient's spine on which the operation will occur, the orientation of the visual marker (and more particularly, of a length dimension, longitudinal axis, or other predetermined dimension or axis of the visual marker) may be assigned as or otherwise correlated with the alignment of that portion of the spine.

While the method 700 relies on an assumption that the visual marker has been properly oriented relative to the anatomical feature, such that the alignment of the visual marker may be assigned as or otherwise correlated with the alignment of the anatomical feature, any risk inherent in making such an assumption is reduced given placement of the visual marker on the patient is likely to be done by a trained medical professional (given that surgeons and operating room staff tend to be trained medical professionals). Moreover, the method 700 beneficially enables an alignment determination to be made without utilizing imaging devices that emit X-rays or other potentially harmful radiation, and in a manner that does not require time-intensive alignment of an imaging device with the patient.

Although the method 700 is described in connection with determining an alignment of an anatomical feature using a visual marker, embodiments of the present disclosure may utilize a visual or other marker as described herein to aid in the detection of any object of interest in a volume of interest, including, for example, a target anatomical feature (e.g., an anatomical feature that will be cut or otherwise modified during the surgical procedure described in the surgical plan), a non-target anatomical feature (e.g., an anatomical feature to be avoided or that might be confused with a target anatomical feature), a non-anatomical object (e.g., a tube, surgical tool, instrument, or other man-made device)). The method 700 may be used, for example, to identify and distinguish target tissue from non-target tissue. In some embodiments, multiple visual markers may be used to aid in identification of a plurality of anatomical or non-anatomical objects, including one or both of target anatomical features and non-target anatomical features.

The present disclosure encompasses embodiments of the method 700 that comprise more or fewer steps than those described above, and/or that comprise executing the steps described above in a different order than described above. The present disclosure also encompasses embodiments of the method 700 that comprise one or more steps other than those described above, including any one or more steps of any of the methods 200, 300, 400, 500, 600, 650, and/or 800 described herein.

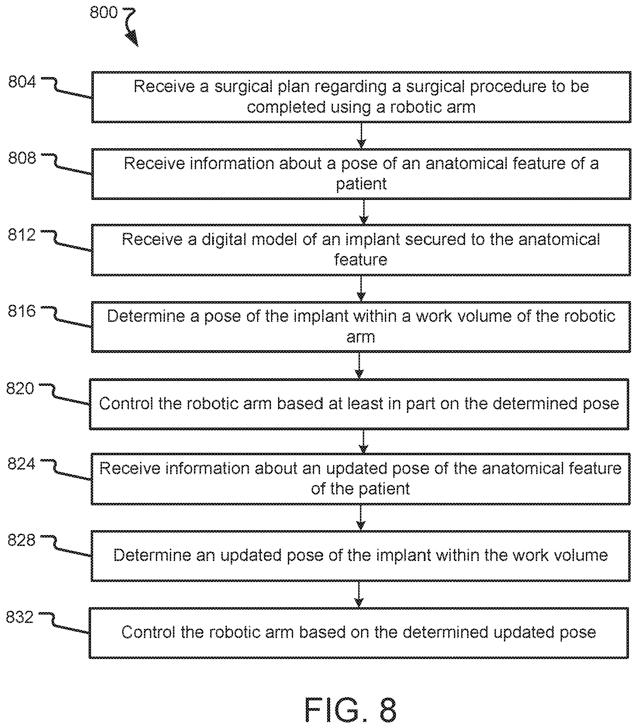

Fig. 8 describes a method 800 for determining a movement of an object during a surgical procedure and controlling a robotic arm accordingly. The method 800 beneficially increases accuracy (by accounting for movement of objects during a surgical procedure), improves patient safety, reduces the time required to complete a surgical procedure (by recognizing and planning for potential conflicts that might otherwise take extra time to resolve), and provides for the efficient completion of a surgical procedure. The method 800 may be carried out, for example, by a processor (e.g., the processor 104) executing instructions stored in a memory (e.g., the memory 116).

The method 800 comprises receiving a surgical plan regarding a surgical procedure to be completed using a robotic arm (step 804). The surgical plan may be, for example, a surgical plan 128, and may be received from a memory such as the memory 116, or from a database such as the database 148, a network (e.g., the cloud 152), or any other source. The surgical plan may be the same as or similar to any other surgical plan described herein.

The method 800 also comprises receiving information about a pose of an anatomical feature of a patient (step 808). The information may be received from a navigation system such as the navigation system 144, or from one or more sensors such as the sensor 132, or from any other source. The information may be, for example, information about a pose (e.g., a position and orientation) of a bone, an organ, an artery, or any other anatomical feature. The information may be in the form of an image or other image data, or may be in the form of one or more coordinates and/or angles, or may be in any other form. The information may be received automatically or in response to a user command (input, for example, via the user interface 112).

The method 800 also comprises receiving a digital model of an implant secured to the anatomical feature (step 812). The implant may be identified, for example, in the surgical plan. The digital model may be, for example, a model 124. The digital model may be received with or via the surgical plan, or may be received from a memory such as the memory 116 or a database such as the database 148. The digital model may be received via a network such as the cloud 152.

Where the anatomical feature is a vertebra and the implant is a screw comprising a screw tower, the digital model may be a digital model of the screw and/or the screw tower. Information about an attachment of the implant to the anatomical feature may be contained within the surgical plan, or received together with or in the same manner (and/or from the same source) as the pose of the anatomical feature of the patient.

The method 800 also comprises determining a pose of the implant within a work volume of the robotic arm. The determining may be based on the information about the pose of the anatomical feature as well as the digital model of the implant. The determining may also be based on the surgical plan. Using information about an attachment of the implant to the anatomical feature (whether from the surgical plan or any other source), the digital model, as well as the information about the pose of the anatomical feature, a pose of the implant may be determined. Thus, for example, if a pose of a vertebra is received in the step 808, and a digital model of a screw tower attached to the vertebra is received in the step 812, then the digital model may be manipulated based on the pose of the vertebra (and, in some embodiments, based on a known relationship between the vertebra and the implant) to determine a pose of the implant. The pose of the implant includes a position and orientation of the implant, and is thus useful for determining, for example, the boundaries of a volume occupied by the implant.

The method 800 also comprises controlling the robotic arm based at least in part on the determined pose (step 820). The step 820 may be the same as or similar to the step 620, described above. For example, the controlling may comprise generating a movement path based on the determined pose of the implant, and then causing the robotic arm to move along the movement path. The controlling may also comprise stopping movement of the robotic arm if the robotic arm is determined to be unacceptably close to the implant, or causing the robotic arm to deviate from a current movement path in order to avoid colliding with or otherwise impacting the implant. The controlling may comprise sending one or more signals to a robot that comprises the robotic arm, which signals cause the robot to move the robotic arm in a particular way. The controlling may also comprise opening a power circuit through which the robot receives power, so as to immediately stop the robot. The controlling may further comprise selectively operating one or more motors in the robotic arm to cause the robotic arm to move along a desired path. Depending on the nature of the surgical procedure, the controlling may comprise moving the robotic arm to avoid the implant, or moving the robotic arm to facilitate interaction between the robotic arm (and/or a tool or instrument held by the robotic arm) and the implant.

The method 800 also comprises receiving information about an updated pose of the anatomical feature of the patient (step 824). The information about the updated pose may be received from the same source as the information about the pose of the anatomical feature as described with respect to the step 808 above, or from a different source. For example, the information about the updated pose may be received from a navigation system such as the navigation system 144, or from one or more sensors such as the sensor 132, or from any other source. The information about the updated pose may be received at a second time after a first time at which the information about the pose was received. In such embodiments, the second time may be one second or less after the first time (e.g., where a video or real-time stream is being used to obtain information about the pose of the anatomical feature of the patient), or more than one second, more than one minute, more than five minutes, more than twenty minutes, or more than one hour after the first time (e.g., where still images or snapshots are being used to monitor the pose of the anatomical feature of the patient). The information about the updated pose may be received in the same manner and via the same path or route as the information about the pose, or in a different manner and/or via a different path.

The method 800 also comprises determining an updated pose of the implant within the work volume (step 828). The determining the updated pose of the implant may be accomplished in the same manner as, or in a similar manner to, the determining the pose of the implant, as described above with respect to the step 816. The updated pose of the implant may be the same as the pose of the implant determined in the step 816 (e.g., where the updated pose of the anatomical feature is the same as the pose of the anatomical feature), or the updated pose of the implant may be different than the pose of the implant determined in the step 816.

The method 800 also comprises controlling the robotic arm based on the determined updated pose (step 832). The controlling the robotic arm based on the determined updated pose may be the same as or substantially similar to the controlling the robotic arm based on the determined pose of the implant in the step 820.

The present disclosure encompasses embodiments of the method 800 that comprise more or fewer steps than those described above, and/or that comprise executing the steps described above in a different order than described above. The present disclosure also encompasses embodiments of the method 800 that comprise one or more steps other than those described above, including any one or more steps of any of the methods 200, 300, 400, 500, 600, 650, and/or 700 described herein.

The foregoing discussion has been presented for purposes of illustration and description. Based on the disclosure here and the examples provided, including with respect to automatic detection of a vertebral endplate rim, persons of ordinary skill in the art will understand how to utilize the systems and methods of the present disclosure to automatically detect other anatomical structures.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

## Claims

1. A surgical robotic system (100), comprising:
a robot (136) comprising a robotic arm (140);
at least one processor (104);
a sensor (132);
a navigation system (144) configured for providing navigation for the robot (136) during an operation and tracking a position of the sensor (132) and of the robot (136); and
a memory (116) storing instructions for execution by the at least one processor (104) that, when executed, cause the at least one processor (104) to:
receive a surgical plan comprising first information about a planned position of at least one object relative to a patient's anatomy and second information about a surgical objective;
receive a digital model corresponding to the at least one object;
receive, from the sensor (132), third information about the surgical environment comprising the at least one object;
identify, based on the third information, an actual position of the at least one object and recognize the at least one object in the third information;
determine, whether the at least one object must be moved or avoided;
based on the determination, identify a needed movement of the at least one object to clear a movement path for the robotic arm (140) or modify the movement path of the robotic arm (140);
in case of modifying the movement path of the robotic arm (140), calculate the movement path for the robotic arm (140) based on the first information, the second information and the digital model; and
control the robotic arm (140) based on the calculated movement path.

2. The surgical robotic system (100) of claim 1, wherein the third information is received at a first time, and the memory (116) stores additional instructions for execution by the at least one processor (104) that, when executed, further cause the at least one processor (104) to:
receive, from the sensor (132) and at a second time after the first time, fourth information about the surgical environment;
determine, based on a comparison of the third information and the fourth information, whether the actual position of the at least one object changed from the first time to the second time; and
control the robotic arm (140) based on the determination.

3. The surgical robotic system (100) of claim 1, wherein the at least one object is an incision.

4. The surgical robotic system (100) of claim 1, wherein the at least one object is a bone or a marker attached to the bone.

5. The surgical robotic system (100) of claim 1, wherein the at least one object is a non-anatomical object.

6. The surgical robotic system (100) of claim 1, wherein the sensor (132) is secured to the robotic arm (140).

## Patentansprüche

1. Chirurgisches Robotersystem (100), umfassend:
einen Roboter (136), umfassend einen Roboterarm (140);
mindestens einen Prozessor (104);
einen Sensor (132);
ein Navigationssystem (144), das zum Bereitstellen einer Navigation für den Roboter (136) während einer Operation und zum Verfolgen einer Position des Sensors (132) und des Roboters (136) konfiguriert ist; und
einen Speicher (116), der Anweisungen für die Ausführung durch den mindestens einen Prozessor (104) speichert, die, wenn sie ausgeführt werden, den mindestens einen Prozessor (104) veranlassen zum:
Empfangen eines Operationsplans, umfassend erste Informationen über eine geplante Position mindestens eines Objekts relativ zu der Anatomie eines Patienten und zweite Informationen über ein Operationsziel;
Empfangen eines digitalen Modells, das dem mindestens einen Objekt entspricht;
Empfangen, von dem Sensor (132), von dritten Informationen über die chirurgische Umgebung, umfassend das mindestens eine Objekt;
Identifizieren, basierend auf den dritten Informationen, einer tatsächlichen Position des mindestens einen Objekts und Erkennen des mindestens einen Objekts in den dritten Informationen;
Bestimmen, ob das mindestens eine Objekt bewegt oder vermieden werden muss;
basierend auf der Bestimmung, Identifizieren einer benötigten Bewegung des mindestens einen Objekts, um einen Bewegungspfad für den Roboterarm (140) freizumachen, oder den Bewegungspfad des Roboterarms (140) zu modifizieren;
im Falle des Modifizierens des Bewegungspfads des Roboterarms (140), Berechnen des Bewegungspfads für den Roboterarm (140) basierend auf den ersten Informationen, den zweiten Informationen und dem digitalen Modell; und
Steuern des Roboterarms (140) basierend auf dem berechneten Bewegungspfad.

2. Chirurgisches Robotersystem (100) nach Anspruch 1, wobei die dritten Informationen zu einem ersten Zeitpunkt erhalten werden, und der Speicher (116) zusätzliche Anweisungen für die Ausführung durch den mindestens einen Prozessor (104) speichert, wenn sie ausgeführt werden, den mindestens einen Prozessor (104) ferner veranlassen zum:
Empfangen, von dem Sensor (132) und zu einem zweiten Zeitpunkt nach dem ersten Zeitpunkt, von vierten Informationen über die chirurgische Umgebung;
Bestimmen, basierend auf einem Vergleich der dritten Informationen und der vierten Informationen, ob sich die tatsächliche Position des mindestens einen Objekts von dem ersten Zeitpunkt zu dem zweiten Zeitpunkt geändert hat; und
Steuern des Roboterarms (140) basierend auf der Bestimmung.

3. Chirurgisches Robotersystem (100) nach Anspruch 1, wobei das mindestens eine Objekt eine Inzision ist.

4. Chirurgisches Robotersystem (100) nach Anspruch 1, wobei das mindestens eine Objekt ein Knochen oder ein an dem Knochen angebrachter Marker ist.

5. Chirurgisches Robotersystem (100) nach Anspruch 1, wobei das mindestens eine Objekt ein nichtanatomisches Objekt ist.

6. Chirurgisches Robotersystem (100) nach Anspruch 1, wobei der Sensor (132) an dem Roboterarm (140) befestigt ist.

## Revendications

1. Système robotique chirurgical (100), comprenant :
un robot (136) comprenant un bras robotique (140) ;
au moins un processeur (104) ;
un capteur (132) ;
un système de navigation (144) configuré pour fournir une navigation pour le robot (136) pendant une opération et suivre une position du capteur (132) et du robot (136) ; et
une mémoire (116) stockant des instructions destinées à être exécutées par l'au moins un processeur (104) qui, lorsqu'elles sont exécutées, amènent l'au moins un processeur (104) à :
recevoir un plan chirurgical comprenant de premières informations concernant une position planifiée d'au moins un objet par rapport à l'anatomie d'un patient et de secondes informations concernant un objectif chirurgical ;
recevoir un modèle numérique correspondant à l'au moins un objet ;
recevoir, du capteur (132), de troisièmes informations concernant l'environnement chirurgical comprenant l'au moins un objet ;
identifier, sur la base de troisièmes informations, une position réelle de l'au moins un objet et reconnaître l'au moins un objet dans les troisièmes informations ;
déterminer si l'au moins un objet doit être déplacé ou évité ;
sur la base de la détermination, identifier un mouvement nécessaire de l'au moins un objet pour dégager une trajectoire de mouvement pour le bras robotique (140) ou modifier la trajectoire de mouvement du bras robotique (140) ;
en cas de modification de la trajectoire de déplacement du bras robotique (140), calculer la trajectoire de déplacement pour le bras robotique (140) sur la base des premières informations, des deuxièmes informations et du modèle numérique ; et
commander le bras robotique (140) sur la base de la trajectoire de mouvement calculée.

2. Système robotique chirurgical (100) selon la revendication 1, dans lequel les troisièmes informations sont reçues à un premier moment, et la mémoire (116) stocke des instructions supplémentaires destinées à être exécutées par l'au moins un processeur (104) qui, lorsqu'elles sont exécutées, amènent en outre l'au moins un processeur (104) à :
recevoir, du capteur (132) et à un second instant après le premier instant, des quatrièmes informations concernant l'environnement chirurgical ;
déterminer, sur la base d'une comparaison des troisièmes informations et des quatrièmes informations, si la position réelle de l'au moins un objet a changé du premier instant au second instant ; et
commander le bras robotique (140) sur la base de la détermination.

3. Système robotique chirurgical (100) selon la revendication 1, dans lequel l'au moins un objet est une incision.

4. Système robotique chirurgical (100) selon la revendication 1, dans lequel l'au moins un objet est un os ou un marqueur fixé à l'os.

5. Système robotique chirurgical (100) selon la revendication 1, dans lequel l'au moins un objet est un objet non anatomique.

6. Système robotique chirurgical (100) selon la revendication 1, dans lequel le capteur (132) est fixé au bras robotique (140).
